# FASCICULE DE BREVET EUROPEEN

(11) **EP 0 516 520 B1**
(45) Date de publication et mention de la délivrance du brevet: **02.01.1997**
(21) Numéro de dépôt: 92401419.4
(22) Date de dépôt: 25.05.1992
(51) Int. Cl.: C07D 211/26, C07D 401/12, A61K 31/445

(54) **Pipéridines disubstituées-1,4, leur préparation et leur application en thérapeutique**
1,4-Disubstituierte Piperidinderivate, ihre Herstellung und therapeutische Verwendung
1,4-Disubstituted piperidines, their preparation and therapeutical application

(30) Priorité: 27.05.1991 FR 9106327
(43) Date de publication de la demande: 02.12.1992
(73) Titulaire: PIERRE FABRE MEDICAMENT, 92100 Boulogne (FR)
(72) Inventeur: Bigg, Denis, F-81100 Castres (FR); Vidaluc, Jean-Louis, F-81100 Castres (FR); Calmel, Francis, F-81100 Castres (FR)
(74) Mandataire: Ahner, Francis

(56) Documents cités:
- EP-A- 0 296 560
- EP-A- 0 392 802
- EP-A- 0 411 615
- EP-A- 0 419 397
- EP-A- 0 468 187
- US-A- 4 057 636
- US-A- 5 006 523
- MEDICINAL CHEMISTRY, 3e édition, 1ere partie, 1970, Wiley-Interscience, New York, A. BURGER, pages 64-80.
- CHEM. SOC. REVIEWS, Vol.18, 1979, pages 563-580.

## Description

La présente invention, réalisée au Centre de Recherche Pierre Fabre, a pour objet de nouvelles pipéridines disubstituées-1,4, leur préparation et leur application en thérapeutique.

Des dérivés de pipéridines complexes, substitués notamment par des groupes dérivés de l'indanonyle et présentant une activité inhibitrice de l'acétylcholinestérase sont décrites dans EP-A-0 296 560.

Les composés de l'invention répondent à la formule générale 1 : 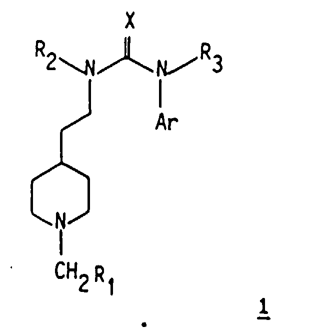 dans laquelle :
- R₁: représente un groupe cycloalkyle en C₅-C₇, un groupe phényle, ou un radical phényle substitué par un groupe alkyle en C₁-C₄, un groupe alkoxy en C₁-C₄, un radical nitro ou un atome d'halogène ;
- R₂ et R₃: représentent, indépendamment l'un de l'autre, un atome d'hydrogène ou un groupe alkyle en C₁-C₄ ;
- X: représente un atome de soufre;
- Ar: représente un groupe pyridyle ou un groupe phényle, substitué ou non par un ou plusieurs substituants choisis parmi un atome d'halogène, un groupe alkyle en C₁-C₄, un groupe alkoxy en C₁-C₄, un groupe acyle en C₁-C₄, un groupe cyano, un groupe nitro, un groupe trifluorométhyle, ou un groupe trifluorométhoxy.

L'invention couvre également les sels des composés de formule générale 1 avec des acides minéraux ou organiques pharmaceutiquement acceptables, à titre d'exemple non limitatif, l'acide employé peut être l'acide chlorhydrique ou l'acide fumarique.

Les composés de formule générale 1 de l'invention peuvent être préparés selon les méthodes décrites ci-dessous.
Les composés de formule générale 1 où R₃ représente un atome d'hydrogène peuvent être préparés par la réaction d'une amine de formule générale 2 avec un isothiocyanate de formule générale 3 selon le schéma suivant : 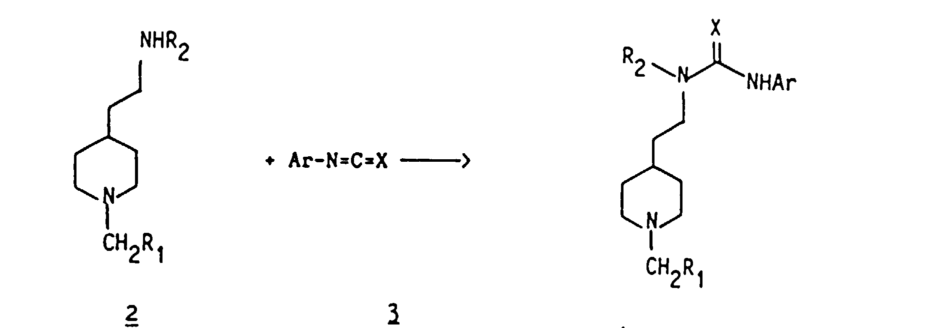 où R₁, R₂ et Ar sont définis comme ci-dessus.

Les amines de départ 2 et les composés de formule générale 3 peuvent être préparés selon des méthodes connues.

La réaction d'un composé de formule générale 2 avec un isothiocyanate de formule 3 (X=S) s'effectue au sein d'un solvant tel qu'un solvant chloré, par exemple le dichlorométhane ou le dichloroéthane, un solvant cétonique, un ester, un éther, ou un alcool. La réaction peut être effectuée à la température ambiante, ou accélérée par simple chauffage.

Les composés de formule générale 1 où R₂ représente un atome d'hydrogène peuvent être préparés par la réaction d'un isothiocyanate de formule générale 4 avec une amine de formule générale 5 selon le schéma suivant : 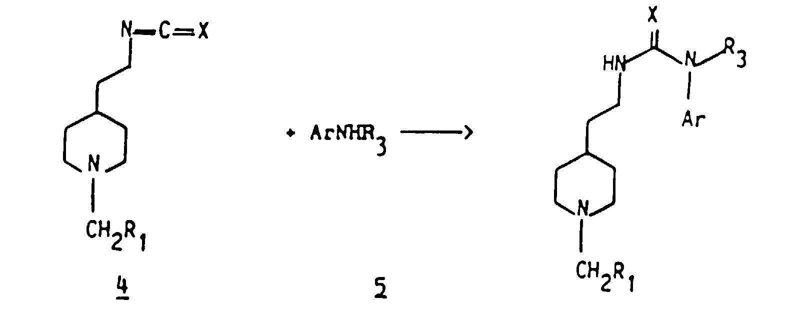 où R₁, R₃ et Ar sont définis comme ci-dessus.

Les hétérocumulènes de formule générale 4 et les amines de formule générale 5 peuvent être préparés selon des méthodes connues.

La réaction d'un isothiocyanate de formule générale 4 (X=S) avec une amine de formule générale 5 s'effectue au sein d'un solvant tel qu'un solvant chloré, par exemple le dichlorométhane ou le dichloroéthane, un solvant cétonique, un ester, un éther ou un alcool. La réaction peut être effectuée à la température ambiante ou accélérée par simple chauffage.

Les exemples suivants illustrent l'invention sans toutefois en limiter la portée.

### Exemple 1

Phényl-1[(benzyl-1 pipéridinyl-4) éthyl-2)-3 thiourée : composé n°1 ; R₁ = Ph, R₂ = R₃ = H, X = S, Ar = Ph.

On ajoute 3,0 ml (25 mmoles) d'isothiocyanate de phényle à une solution de 5,0 g (22,9 mmoles) de (benzyl-1 pipéridinyl-4)-2 éthylamine dans 50 ml de dichlorométhane sous agitation à la température ambiante. Après une heure le solvant est évaporé et le résidu huileux ambré repris par l'éther éthylique. On obtient 7,3 g (90 %) du composé 1 sous forme de cristaux blancs.
PF° : 113-115°C.

Le tableau 1 ci-après résume les principaux produits synthétisés qui illustrent l'invention sans toutefois en limiter la portée.

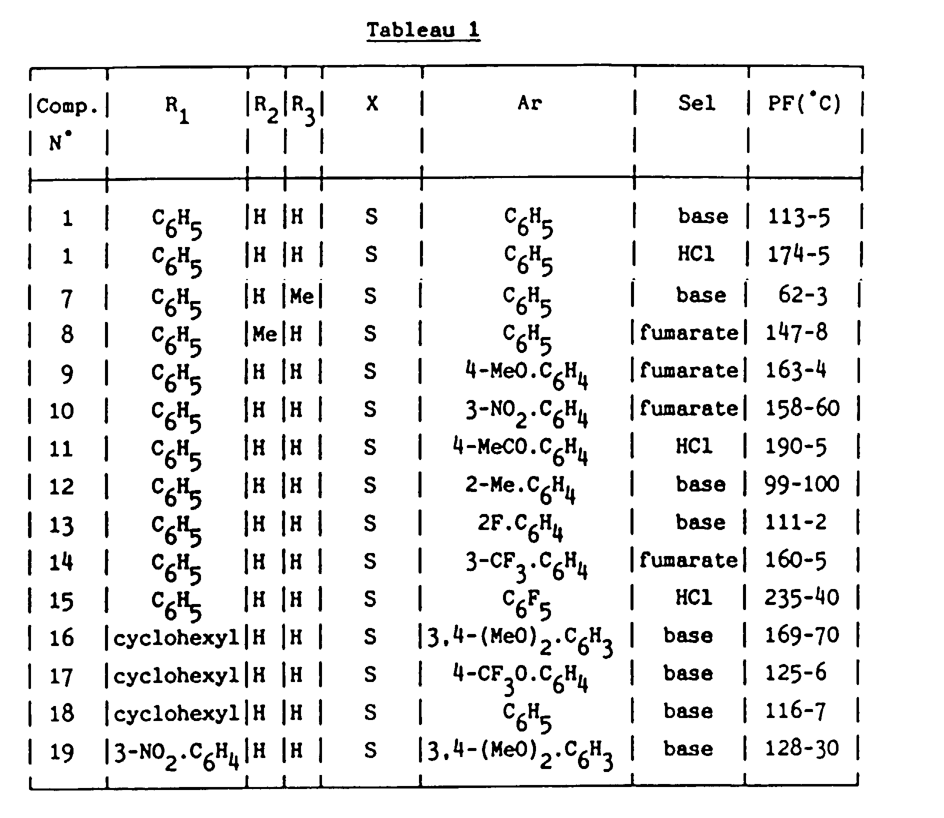

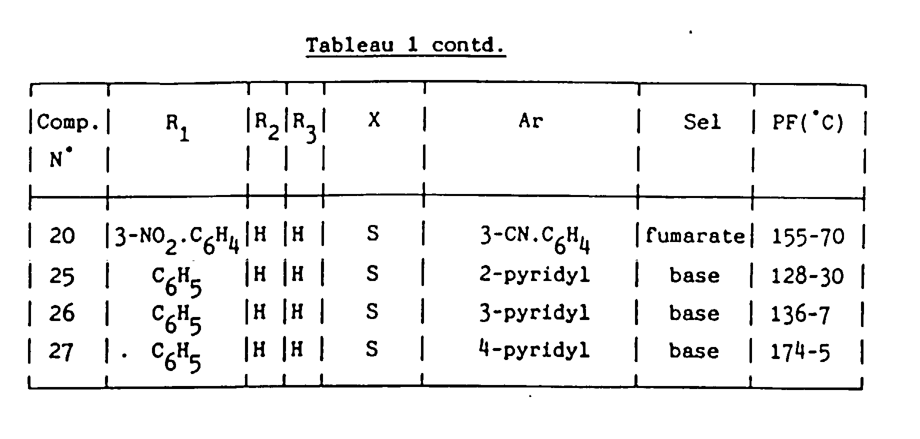

Les composés de l'invention ont été soumis à des essais pharmacologiques qui ont montré leur intérêt en tant qu'inhibiteurs de l'acétylcholinestérase.
A cet effet, les composés ont été étudiés selon la méthode décrite par G.L. Ellman et al., Biochem. Pharmacol., 7, 88-95 (1961).

Les résultats obtenus sur certains composés de l'invention sont reportés, à titre d'exemple, dans le tableau 2.

**Tableau 2**

| **Inhibition de l'activité de l'acétylcholinestérase** | |
|---|---|
| Composé n° | IC₅₀ (µM) |
| 2 | 0,32 |
| 9 | 0,15 |
| 25 | 0,02 |
| Tacrine | 0,12 |

Les composés de l'invention sont des inhibiteurs de l'acétylcholinestérase ; aussi, ils peuvent être utiles dans le traitement des maladies telles que la myasthénie, les troubles de la mémoire, les démences, telles que les démences séniles ou la maladie d'Alzheimer.

Les préparations pharmaceutiques contenant ces principes actifs peuvent être mises en forme pour l'administration par voie orale, rectale, parentérale ou locale, par exemple sous la forme de capsules, comprimés, granulés, gélules, solutés liquides, sirops ou suspensions buvables, et contenir les excipients appropriés.

Il est également possible d'y associer d'autres principes actifs pharmaceutiquement et thérapeutiquement acceptables.

## Revendications

1. Pipéridines disubstituées-1,4 correspondant à la formule générale 1 : 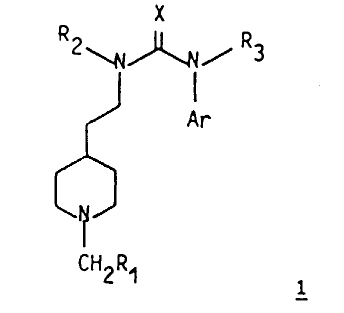 dans laquelle :
R₁ représente un groupe cycloalkyle en C₅-C₇, un groupe phényle, ou un radical phényle substitué par un groupe alkyle en C₁-C₄, un groupe alkoxy en C₁-C₄, un radical nitro ou un atome d'halogène ;
R₂ et R₃ représentent, indépendamment l'un de l'autre, un atome d'hydrogène ou un groupe alkyle en C₁-C₄ ;
X représente un atome de soufre;
Ar représente un groupe pyridyle ou un groupe phényle, substitué ou non par un ou plusieurs substituants choisis parmi un atome d'halogène, un groupe alkyle en C₁-C₄, un groupe alkoxy en C₁-C₄, un groupe acyle en C₁-C₄, un groupe cyano, un groupe nitro, un groupe trifluorométhyle, ou un groupe trifluorométhoxy;
ainsi que les sels organiques ou minéraux thérapeutiquement acceptables de ces molécules.

2. Composés de formule générale 1 selon la revendication 1 caractérisés par le fait qu'ils sont choisis parmi :
- Phényl-1 [(benzyl-1 pipéridinyl-4)éthyl-2]-3 thiourée
- Méthyl-1 phényl-1 [(benzyl-1 pipéridinyl-4)éthyl-2]-3 thiourée
- Phényl-1 [(benzyl-1 pipéridinyl-4)éthyl-2]-3 méthyl-3 thiourée
- (Méthoxy-4 phényl)-1 [(benzyl-1 pipéridinyl-4)éthyl-2]-3 thiourée
- (Nitro-3 phényl)-1 [(benzyl-1 pipéridinyl-4)éthyl-2]-3 thiourée
- (Acétyl-4 phényl)-1 [(benzyl-1 pipéridinyl-4)éthyl-2]-3 thiourée
- (Acétyl-2 phényl)-1 [(benzyl-1 pipéridinyl-4)éthyl-2]-3 thiourée
- (Fluoro-2 phényl)-1 [(benzy1-1 pipéridinyl-4)éthyl-2]-3 thiourée
- (Trifluorométhyl-3 phényl)-1 [(benzyl-1 pipéridinyl-4)éthyl-2]-3 thiourée
- Pentafluorophényl-1 [(benzyl-1 pipéridinyl-4)éthyl-2]-3 thiourée
- (Diméthoxy-3,4 phényl)-1 [(cyc1ohexylméthy1-1 pipéridinyl-4) éthyl-2]-3 thiourée
- (Trifluorométhoxy-4 phényl)-1 [(cyclohexylméthyl-1 pipéridinyl-4) éthyl-2]-3 thiourée
- Phényl-1 [(cyclohexylméthyl-1 pipéridinyl-4)éthyl-2]-3 thiourée
- (Diméthoxy-3,4 phényl)-1 [{(nitro-3 phénylaéthyl)-1 pipéridinyl-4}éthyl-2]-3 thiourée
- (Cyano-3 phényl)-1 [{(nitro-3 phénylméthyl)-1 pipéridinyl-4} éthyl-2]-3 thiourée
- (Pyridyl-2)-1 [(benzyl-1 pipéridinyl-4)éthyl-2]-3 thiourée
- (Pyridyl-3)-1 [(benzyl-1 pipéridinyl-4)éthyl-2]-3 thiourée
- (Pyridyl-4)-1 [(benzyl-1 pipéridinyl-4)éthyl-2]-3 thiourée

3. Procédé de préparation de composés selon les revendications 1 et 2 caractérisé en ce que l'on fait réagir une amine de formule générale 2 avec un isothiocyanate de formule générale 3 : 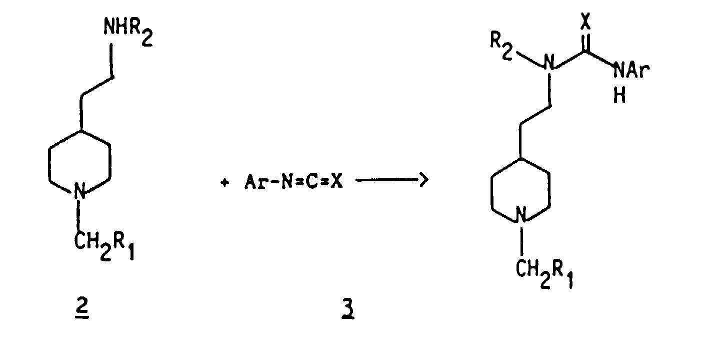 où R₁, R₂ et Ar sont définis comme ci-dessus et X représente un atome de soufre.

4. Procédé de préparation de composés selon la revendication 3 caractérisé en ce que la réaction entre une amine de formule générale 2 et un isothiocyanate de formule générale 3, où X représente un atome de soufre, s'effectue en solution.

5. Procédé selon la revendication 4, caractérisé en ce que le solvant employé est choisi parmi un solvant chloré tel que le dichlorométhane ou le dichloroéthane, un solvant cétonique, un ester, un éther, ou un alcool.

6. Procédé de préparation de composés selon les revendications 1 et 2 caractérisé en ce que l'on fait réagir un isothiocyanate de formule générale 4 avec une amine de formule générale 5 : 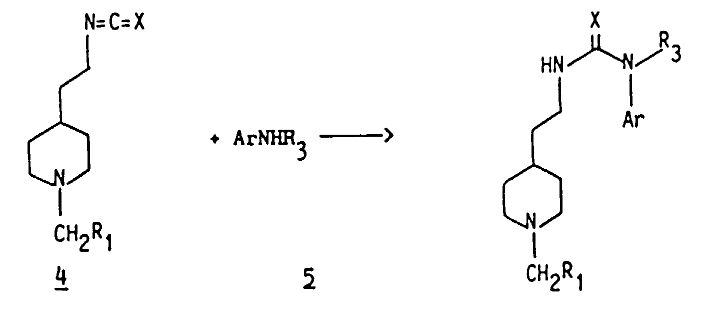 où R₁, R₃ et Ar sont définis comme ci-dessus et X représente un atome de source.

7. Procédé de préparation de composés selon la revendication 6 caractérisé en ce que la réaction entre un isothiocyanate de formule générale 4, où X représente un atome de source et une amine de formule générale 5 s'effectue en solution.

8. Procédé selon la revendication 7, caractérisé en ce que le solvant employé est choisi parmi un solvant chloré tel que le dichlorométhane ou le dichloroéthane, un solvant cétonique, un ester, un éther, ou un alcool.

9. A titre de médicaments utiles, par exemple, dans le traitement de la myasthénie, les troubles de la mémoire, les démences, telles que les démences séniles ou la maladie d'Alzheimer, les composés définis selon l'une des revendications 1 et 2.

10. Composition pharmaceutique caractérisée en ce qu'elle contient un composé de l'une des revendications 1 et 2 en association avec tout excipient approprié.

## Patentansprüche

1. Disubstituierte 1,4-Piperidine entsprechend der allgemeinen Formel 1: 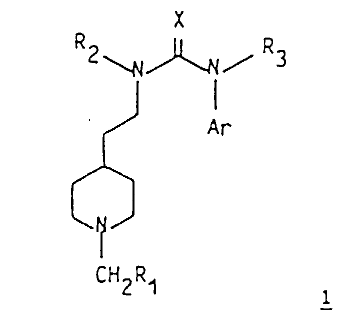 wobei:
R₁ eine C₅-C₇-Cycloalkylgruppe, eine Phenylgruppe oder ein durch eine C₁-C₄-Alkylgruppe, eine C₁-C₄-Alkoxygruppe, eine Nitrogruppe oder ein Halogenatom substituierte Phenylgruppe darstellt;
R₂ und R₃ unabhängig voneinander ein Wasserstoffatom oder eine C₁-C₄-Alkylgruppe darstellen;
X ein Schwefelatom darstellt;
Ar eine Pyridylgruppe oder Phenylgruppe darstellt, gegebenenfalls substituiert durch einen oder mehrere Substituenten, ausgewählt aus einem Halogenatom, einer C₁-C₄-Alkylgruppe, einer C₁-C₄-Alkoxygruppe, einer C₁-C₄-Acylgruppe, einer Cyanogruppe, einer Nitrogruppe, einer Trifluormethylgruppe oder einer Trifluormethoxygruppe;
sowie die therapeutisch verträglichen organischen oder mineralischen Salze dieser Moleküle.

2. Verbindungen der allgemeinen Formel 1 nach Anspruch 1, dadurch charakterisiert, daß sie ausgewählt werden aus:
- 1-Phenyl-3-[1-benzyl-4-piperidinyl)-2-ethyl]thioharnstoff
- 1-Methyl-1-phenyl-3-[1-benzyl-4-piperidinyl)-2-ethyl]thioharnstoff
- 1-Phenyl-3-[(1-benzyl-4-piperidinyl)-2-ethyl]-3-methylthioharnstoff
- 1-(4-Methyoxyphenyl)-3-[(1-benzyl-4-piperidinyl)-2-ethyl]thioharnstoff
- 1-(3-Nitrophenyl)-3-[(1-benzyl-4-piperidinyl)-2-ethyl]thioharnstoff
- 1-(4-Acetylphenyl)-3-[(1-benzyl-4-piperidinyl)-2-ethyl]-thioharnstoff
- 1-(2-Methylphenyl)-3-[(1-benzyl-4-piperidinyl)-2-ethyl]-thioharnstoff
- 1-(2-Fluorphenyl)-3-[(1-benzyl-4-piperidinyl)-2-ethyl]thioharnstoff
- 1-(3-Trifluormethylphenyl)-3-[(1-benzyl-4-piperidinyl)-2-ethyl]-thioharnstoff
- 1-Pentafluorphenyl-3-[(1-benzyl-4-piperidinyl)-2-ethyl]-thioharnstoff
- 1-(3,4-Dimethoxyphenyl)-3-[(1-cyclohexylmethyl-4-piperidinyl)-2-ethyl]-thioharnstoff
- 1-(4-Trifluormethoxyphenyl)-3-[(1-cyclohexylmethyl-4-piperidinyl)-2-ethyl]-thioharnstoff
- 1-Phenyl-3-[(1-cyclohexylmethyl-4-piperidinyl)-2-ethyl]-thioharnstoff
- 1-(3,4-Dimethoxyphenyl)-3-[{1-(3-nitrophenylmethyl)-4-piperidinyl}-2-ethyl]-thioharnstoff
- 1-(3-Cycanophenyl)-3-[{1-(3-nitrophenylmethyl)-4-piperidinyl}-2-ethyl]-thioharnstoff
- 1-(2-Pyridyl)-3-[(1-benzyl-4-piperidinyl)-2-ethyl]thioharnstoff
- 1-(3-Pyridyl)-3-[(1-benzyl-4-piperidinyl)-2-ethyl]thioharnstoff
- 1-(4-Pyridyl)-3-[(1-benzyl-4-piperidinyl)-2-ethyl]thioharnstoff

3. Verfahren zur Herstellung von Verbindungen nach Ansprüchen 1 und 2, dadurch charakterisiert, daß man ein Amin der allgemeinen Formel 2 mit einem Isothiocyanat der allgemeinen Formel 3 umsetzt: 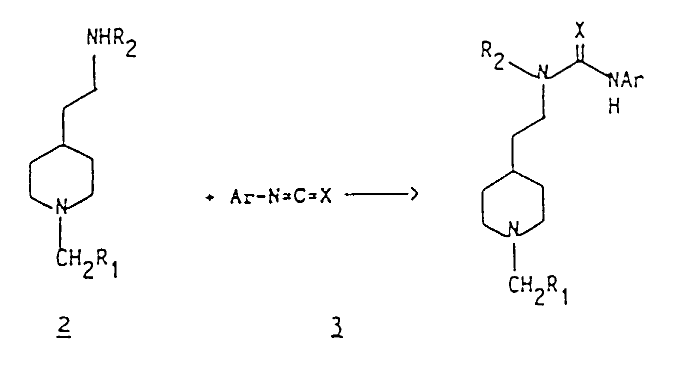 wobei R₁, R₂ und Ar wie oben definiert sind und X ein Schwefelatom darstellt.

4. Verfahren zur Herstellung der Verbindungen nach Anspruch 3, dadurch charakterisiert, daß die Reaktion zwischen einem Amin der allgemeinen Formel 2 und einem Isothiocyanat der allgemeinen Formel 3, wobei X ein Schwefelatom darstellt, in Lösung erfolgt.

5. Verfahren nach Anspruch 4, dadurch charakterisiert, daß das verwendete Lösungsmittel ausgewählt wird aus einem chlorierten Lösungsmittel wie Dichlormethan oder Dichlorethan, einem Ketonlösungsmittel, einem Ester, einem Ether oder einem Alkohol.

6. Verfahren zur Herstellung der Verbindungen nach den Ansprüchen 1 und 2, dadurch charakterisiert, daß man ein Isothiocyanat der allgemeinen Formel 4 mit einem Amin der allgemeinen Formel 5 umsetzt: 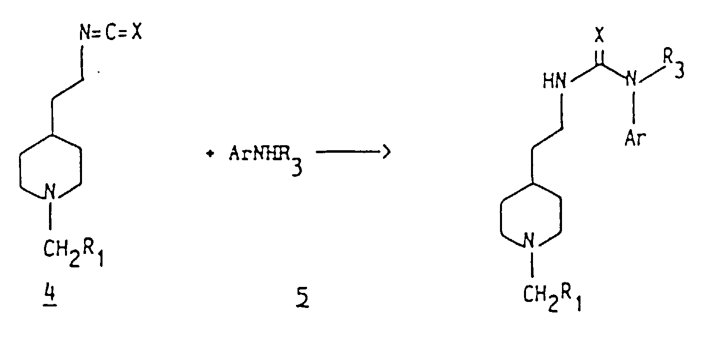 wobei R₁, R₃ und Ar wie oben definiert sind und X ein Schwefelatom darstellt.

7. Verfahren zur Herstellung der Verbindungen nach Anspruch 6, dadurch charakterisiert, daß die Reaktion zwischen einem Isothiocyanat der allgemeinen Formel 4, wobei X ein Schwefelatom darstellt, und einem Amin der allgemeinen Formel 5 in Lösung erfolgt.

8. Verfahren nach Anspruch 7, dadurch charakterisiert, daß das verwendete Lösungsmittel ausgewählt wird aus einem chlorierten Lösungsmittel wie Dichlormethan oder Dichlorethan, einem Ketonlösungsmittel, einem Ester, einem Ether oder einem Alkohol.

9. Die nach einem der Ansprüche 1 und 2 definierten Verbindungen zur Verwendung als Medikamente, beispielsweise für die Behandlung von Myasthenie, Gedächtnisstörungen, Demenzen, wie senilen Demenzen oder Alzheimer-Krankheit.

10. Pharmazeutische Zusammensetzung, dadurch charakterisiert, daß sie eine Verbindung nach einem der Ansprüche 1 und 2 in Verbindung mit jedem geeigneten Excipienten enthält.

## Claims

1. 1,4-Disubstituted piperidines corresponding to the general formula 1: 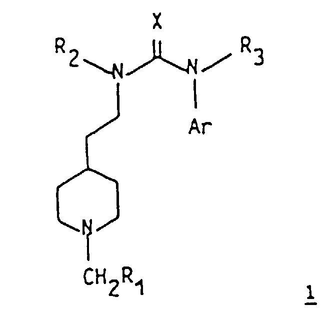 in which:
R₁ represents a C₅-C₇ cycloalkyl group, a phenyl group or a phenyl radical substituted by a C₁-C₄ alkyl group, a C₁-C₄ alkoxy group, a nitro radical or a halogen atom;
R₂ and R₃ represent, independently of each other, a hydrogen atom or a C₁-C₄ alkyl group;
X represents a sulphur atom;
Ar represents a pyridyl group or a phenyl group, optionally substituted by one or more substituents chosen from a halogen atom, a C₁-C₄ alkyl group, a C₁-C₄ alkoxy group, a C₁-C₄ acyl group, a cyano group, a nitro group, a trifluoromethyl group or a trifluoromethoxy group;
as well as the therapeutically acceptable organic or inorganic salts of these molecules.

2. Compounds of general formula 1 according to Claim 1, characterized in that they are chosen from:
- 1-Phenyl-3-[2-(1-benzyl-4-piperidyl)ethyl]thiourea
- 1-Methyl-1-phenyl-3-[2-(1-benzyl-4-piperidyl)ethyl]-thiourea
- 1-Phenyl-3-[2-(1-benzyl-4-piperidyl)ethyl]-3-methyl-thiourea
- 1-(4-Methoxyphenyl)-3-[2-(1-benzyl-4-piperidyl)ethyl]-thiourea
- 1-(3-Nitrophenyl)-3-[2-(1-benzyl-4-piperidyl)ethyl]-thiourea
- 1-(4-Acetylphenyl)-3-[2-(1-benzyl-4-piperidyl)ethyl]thiourea
- 1-(2-Methylphenyl)-3-[2-(1-benzyl-4-piperidyl)ethyl]thiourea
- 1-(2-Fluorophenyl)-3-[2-(1-benzyl-4-piperidyl)ethyl]thiourea
- 1-(3-Trifluoromethylphenyl)-3-[2-(1-benzyl-4-piperidyl) ethyl] thiourea
- 1-Pentafluorophenyl-3-[2-(1-benzyl-4-piperidyl)ethyl]thiourea
- 1-(3,4-Dimethoxyphenyl)-3-[2-(1-cyclohexylmethyl-4-piperidyl)ethyl]thiourea -1-(4-Trifluoromethoxyphenyl)-3-[2-(1-cyclohexylmethyl-4-piperidyl)ethyl]thiourea
- 1-Phenyl-3-[2-(1-cyclohexylmethyl-4-piperidyl)ethyl]thiourea
- 1-(3,4-Dimethoxyphenyl)-3-[2-{1-(3-nitrophenylmethyl)-4-piperidyl}ethyl]thiourea
- 1-(3-Cyanophenyl)-3-[2-{1-(3-nitrophenylmethyl)-4-piperidyl}ethyl]thiourea
- 1-(2-Pyridyl)-3-[2-(1-benzyl-4-piperidyl)ethyl]thiourea
- 1-(3-Pyridyl)-3-[2-(1-benzyl-4-piperidyl)ethyl]thiourea
- 1-(4-Pyridyl)-3-[2-(1-benzyl-4-piperidyl)ethyl]thiourea.

3. Method of preparation of compounds according to Claims 1 and 2, characterized in that an amine of general formula 2 is reacted with an isothiocyanate of general formula 3: 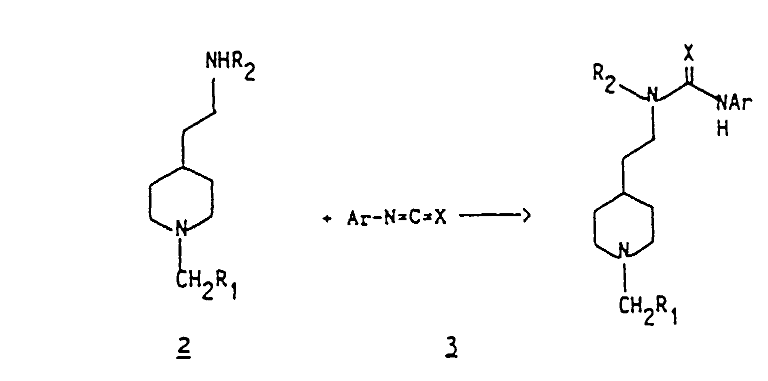 where R₁, R₂ and Ar are defined as above and X represents a sulphur atom.

4. Process of preparation of compounds according to Claim 3, characterized in that the reaction between an amine of general formula 2 and an isothiocyanate of general formula 3, where X represents a sulphur atom, is carried out in solution.

5. Process according to Claim 4, characterized in that the solvent employed is chosen from a chlorinated solvent, such as dichloromethane or dichloroethane, a ketone solvent, an ester, an ether or an alcohol.

6. Method of preparation of compounds according to Claims 1 and 2, characterized in that an isothiocyanate of general formula 4 is reacted with an amine of general formula 5: 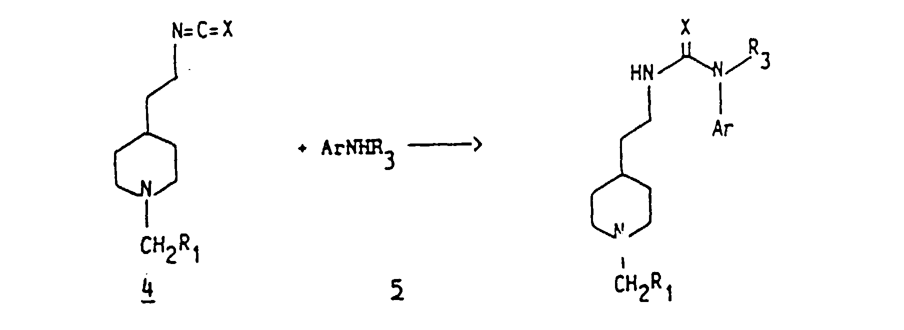 where R₁, R₃ and Ar are defined as above and X represents a sulphur atom.

7. Process of preparation of compounds according to Claim 6, characterized in that the reaction between an isothiocyanate of general formula 4, where X represents a sulphur atom, and an amine of general formula 5 is carried out in solution.

8. Process according to Claim 7, characterized in that the solvent employed is chosen from a chlorinated solvent, such as dichloromethane or dichloroethane, a ketone solvent, an ester, an ether or an alcohol.

9. As medicinal products useful, for example, in the treatment of myasthenia, memory disorders or dementias, such as senile dementias or Alzheimer's disease, the compounds defined according to either of Claims 1 and 2.

10. Pharmaceutical composition, characterized in that it contains a compound of either of Claims 1 and 2 in combination with any appropriate excipient.
